# EUROPEAN PATENT SPECIFICATION

(11) **EP 3 922 314 B1**
(45) Date of publication and mention of the grant of the patent: **20.03.2024**
(21) Application number: 20751967.9
(22) Date of filing: 17.01.2020
(51) Int. Cl.: A61Q 9/04, A61K 8/92, C09D 11/50, A61K 8/31, A61K 8/81

(54) **DEPILATORY PRODUCT WITH THERMOCHROMIC EFFECT**
ENTHAARUNGSPRODUKT MIT THERMOCHROMER WIRKUNG
PRODUIT DÉPILATOIRE À EFFET THERMOCHROME

(30) Priority: 04.02.2019 ES 201930173 U
(43) Date of publication of application: 15.12.2021
(73) Proprietor: Viokox, S.A., 46220 Picassent Valencia (ES)
(72) Inventor: LÓPEZ ALCAY, Eduardo, 46220 Picassent (Valencia) (ES)
(74) Representative: Ungria López, Javier
(86) International application number: PCT/ES2020/070032
(87) International publication number: WO 2020/161372

(56) References cited:
- ES-U- 1 225 956
- FR-A1- 2 794 007
- FR-A1- 2 802 067
- US-B1- 6 174 319

## Description

### Object and technical field of the invention

The invention falls within the field of cosmetics, specifically depilation, and it is aimed at a depilatory composition with thermochromic properties.

### Background of the invention

Very different products are known in the field of depilation to remove superfluous body hair, other than shaving. A type of composition, the most common, requires initial heating before being applied to the skin in a generally molten state, being known as hot waxes. Once applied, it is allowed to solidify before being removed from the skin along with the unwanted hair. This is known in the technical field as epilation, since the hair is pulled from the skin at the root (patent ES2442918 T3). They can also be found in prior art, warm waxes being of common use, wherein there is also a previous heating but at a lower temperature and bands of wax that are spread and applied in the area to be depilated, that use a support material to facilitate the removal thereof (international application WO9730608 A1; ES2123430 B1).

Some of the most innovative compositions are in the form of an emulsion or cream, and can be applied to the skin, at room temperature. The cream includes a substance that degrades the keratin in the hair. Conventionally, said compositions are applied to the skin in areas where unwanted hair is present, leaving them in that area for a predetermined time to allow the keratin of the hair to degrade. Lastly, the composition along with the degraded hair is removed from the skin, usually with an instrument such as a sponge or spatula that drags it. Such compositions are known in the art as depilatory creams (patent EP2355788 B1).

The compositions of depilatory waxes, that need pre-heating, can cause burn problems if the necessary precaution is not taken and the instructions for use are not properly followed, especially in warm and hot waxes.

The problem with these waxes is usually caused because the consumer does not have any reference of the temperature reached by the wax until it is used on the body, causing the burns. Thus, the products that exist on the market either do not indicate to the consumer the change in temperature of the product to be applied or only indicate when the use or non-use temperature has been reached through the container or applicator thereof. However, they do not indicate the temperature at which it should not be used due to over or under heating. An example is found in patent ES2189990, wherein a container for depilatory wax is described that includes an applicator with a thermochromic compound to indicate the application temperature, which changes colour between 40 °C and 60 °C. However, the invention described in ES2189990 exhibits limitations: within the waxes that need heating, there are fat-soluble waxes and water-soluble waxes, and the applicator with a thermochromic compound does not work properly for the former, since it does not allow to correctly visualise the change of the pigment in repeated uses due to the wax film that remains on the applicator during use.

It is evident that the depilatories that carry the most risk are hot waxes, since they require more heating than the rest of the depilatories, followed by the warm waxes.

To solve the problems detected in the state of the art, and avoid possible burns with these depilatory products, a depilatory product has been developed that indicates to the consumer the moment in which they can make good use of the same by simple observation and in which it is not recommended due to being at unsuitable temperature conditions, either low or very high. The depilatory product proposed herein makes this information available to the user by means of a triple change in colour:
- At the origin thereof, the product cannot be used and the colour is observed at room temperature;
- At the first colour change due to the change in one of the pigments, the product is suitable for use; and
- When certain areas begin to appear in which the colour changes to a different one due to the change in colour of a second pigment, the product is too hot and there is a risk of burns, so it is advisable not to use until it is tempered.

### General description of the invention

The present invention relates to a depilatory composition, comprising at least one material with thermochromic properties (colouring type), containing in turn two or more reversible thermochromic or thermosensitive compositions (pigments), the first at a concentration comprised between 0.1-8 % and the second at a concentration comprised between 0.05 %-8 % by weight of the total weight of the thermochromic material, both being heat-sensitive at a temperature above 40 °C (still cold for the application of the wax) but different, in such a way that the first is heat-sensitive in a lower temperature range than the second.

Thanks to the thermochromic properties of the thermochromic material that is contained in the depilatory composition, this shows the ability to change colour according to the temperature applied to it, once heated. The purpose of the change in colour is to warn the consumer of the temperature that the product acquires when heated, such that it is possible to check the temperature thereof by simple observation to know if it is ideal for the use and/or application of the same. It is evident that the presence of the thermochromic material does not alter the depilatory properties of the composition.

The depilatory composition is preferably a warm or hot depilatory wax. Thus, it should be understood that the pigments adapt to the product, in other words, depending on the temperature suitable for use thereof or the excess temperature for each type of wax used, the composition of the thermochromic material is adjusted according to the temperature of use.

When the present specification indicates that the pigment or thermochromic composition is heat-sensitive at a certain temperature, it means that this pigment changes the colour thereof from the original colouring at room temperature to another colouring, the colouring thereof even completely disappearing, by stimulation at the indicated temperature. By using (at least) one thermochromic or colouring composition with two heat-sensitive compositions or pigments, the depilatory product can show at least three colours depending on the temperature applied in heating, namely: 1) the colour formed by the base colour itself plus that of the colouring formed by the two thermochromic pigments at room temperature (room temperature being understood as comprised between 20 °C - 30 °C); 2) a characteristic colour after the disappearance or modification of the colour of one of the thermochromic pigments when a temperature is applied that is suitable for applying the wax; and 3) a characteristic colour after the disappearance or modification of the colour of the second of the thermochromic pigments when a temperature is applied that is too high for applying the wax.

Preferably, the upper temperature limit at which the first pigment changes colour and reaches the maximum level of said change (the acquired colour is homogeneous) coincides with the lower temperature limit at which the second pigment begins to change colour.

The first pigment, which is the one that changes colour at a temperature lower than the second, responds to temperatures equal to or higher than 40 °C. A suitable temperature for applying the depilatory composition is understood to be that comprised between 58 °C-64 °C of the composition itself, preferably between 61 °C- 63 °C. When the temperature of the depilatory composition is equal to or higher than 65 °C (preferably higher than 70 °C), it is considered too hot to use, in such a way that the second pigment or thermochromic composition is sensitive (changes colour or the colour disappears) at this temperature, warning of said danger.

In a particular embodiment, the depilatory composition is purple at room temperature; the colour of the first pigment disappears and turns pink when the temperature is comprised between 60-65 °C and the colour of the second pigment disappears and turns white (base colour) when the temperature is equal to or higher than 65 °C, preferably higher than 70 °C.

Preferably, the thermochromic or colouring material contains at least one polymer or copolymer, such as polyethylene in the most preferred case of all. This polymer or copolymer is used to complete the formulation of the thermochromic material at 100% by weight, together with the thermochromic compositions or pigments.

Preferably, the depilatory composition contains the at least one thermochromic or colouring material in an amount of between 2% and 4% of the total weight of the composition.

In a preferred example that serves to illustrate the invention in a nonlimiting way, the depilatory composition is a hot wax that can have the following formulation:

| **Ingredients** | **% wt/wt** |
|---|---|
| Glyceryl resinate | 45-67% |
| Microcrystalline wax | 10-30% |
| Solubiliser | 5-8% |
| Extract | 2-6% |

| **Ingredients** | **% wtlwt** |
|---|---|
| Beeswax | 2-6% |
| Rubber | 3-5% |
| Thermochromic colourings (Ct): | 2-4% |
| (First thermochromic pigment) | (0.1-8% by weight of Ct) |
| (Second thermochromic pigment) | (0.05-8% by weight of Ct) |
| (Polyethylene) | (csp. 100 by weight of Ct) |
| Cosmetic ingredient | 1.0-2% |
| Essence | 0.25-1.0% |

The invention is further illustrated below, developing this preceding example.

### Examples

### Example 1: A depilatory composition was prepared, which is a hot wax, with the following ingredients:

| **Ingredients** | **% wtlwt** |
|---|---|
| Glyceryl resinate | 56 % |
| Microcrystalline wax | 20 % |
| Solubiliser | 6.5 % |
| Extract | 4.5 % |
| Beeswax | 4 % |
| Rubber | 4 % |
| Thermochromic colourings (Ct): | 3 % |
| (First thermochromic pigment) | (0.1-8% by weight of Ct) |
| (Second thermochromic pigment) | (0.05-8% by weight of Ct) |
| (Polyethylene) | (csp. 100 by weight of Ct) |
| Cosmetic ingredient | 1.5 % |
| Essence | 0.5 % |

The composition is manufactured as follows:
The waxes are melted at 100 °C. Then, the resinate and the rubber are added and melted at 125 °C. Once all is melted and homogeneous, it is refrigerated to 85 °C and the rest of the ingredients are added to the mix (the solubiliser, the extract, the thermochromic colourings in pellet form, the cosmetic ingredient and the essence) to form the final depilatory composition. 15 minutes after the addition, the composition will be ready for packaging.

## Claims

1. A depilatory composition **characterised in that** it comprises at least one thermochromic material containing two or more reversible thermochromic compositions, the first at a concentration comprised between 0.1 and 8 %, and the second at a concentration comprised between 0.05 and 8 % by weight of the total weight of the thermochromic material, both being heat-sensitive at a temperature higher than 40°C but different from each other, in such a way that the first is heat-sensitive in a lower temperature range than the second.

2. The depilatory composition according to claim 1, which is a warm or hot depilatory wax.

3. The depilatory composition according to any one of the preceding claims, wherein the first reversible thermochromic composition is contained in the thermochromic material at a concentration comprised between 0.5 and 5% and the second thermochromic composition at a concentration of 0.1% -5% by weight of the total weight of the thermochromic material.

4. The depilatory composition according to any one of the preceding claims, wherein the upper temperature limit at which the first thermochromic composition completely changes colour and reaches the maximum level of said change, causing a homogeneous colour in the depilatory composition, coincides with the lower temperature limit at which the second thermochromic composition begins to change colour.

5. The depilatory composition according to any one of the preceding claims, wherein the first thermochromic composition changes colour at a temperature comprised between 40 °C and 65 °C, while the second thermochromic composition changes colour at a temperature comprised between 65 °C and 100 °C.

6. The depilatory composition according to any one of the preceding claims, which is purple at room temperature; pink at a temperature comprised between 60-65 °C when the colour of the first thermochromic composition disappears; and
white at a temperature equal to or higher than 65 °C, when the colour of the second depilatory composition disappears.

7. The depilatory composition according to any one of the preceding claims, wherein the thermochromic material contains at least one polymer or one copolymer, to complete the formulation of the thermochromic material at 100% by weight thereof.

8. The depilatory composition of the preceding claim, wherein the copolymer is polyethylene.

9. The depilatory composition according to any one of the preceding claims, containing the at least one thermochromic material in an amount comprised between 2% and 4% of the total weight of the composition.

10. The depilatory composition according to any one of the preceding claims, which is a hot wax that can have the following formulation, in percentage by weight of the total weight of the composition:
- Glyceryl resinate: 45-67%
- Microcrystalline wax: 10-30%
- Solubiliser: 5-8%
- Extract: 2-6%
- Beeswax: 2-6%
- Rubber: 3-5%
- Cosmetic ingredient: 1.0-2%
- Essence: 0.25-1.0%
- Thermochromic colouring materials: 2-4%
∘ First thermochromic pigment: 0.1-8% by weight of the total weight of colouring material
∘ Second thermochromic pigment: 0.05-8% by weight of the total weight of colouring material
∘ Polyethylene: csp. 100 by weight of total weight of colouring material.

## Patentansprüche

1. Enthaarungsmittel, **dadurch gekennzeichnet, dass** es mindestens ein thermochromes Material umfasst, das zwei oder mehr reversible thermochrome Zusammensetzungen enthält, wobei die erste in einer Konzentration zwischen 0,1 und 8 % und die zweite in einer Konzentration zwischen 0,05 und 8 Gew.-% des Gesamtgewichts des thermochromen Materials enthalten ist, wobei beide bei einer Temperatur von mehr als 40°C wärmeempfindlich, aber voneinander verschieden sind, derart, dass die erste in einem niedrigeren Temperaturbereich wärmeempfindlich ist als die zweite.

2. Enthaarungsmittel nach Anspruch 1, bei dem es sich um ein warmes oder heißes Enthaarungswachs handelt.

3. Enthaarungsmittel nach einem der vorhergehenden Ansprüche, bei dem die erste reversible thermochrome Zusammensetzung in dem thermochromen Material in einer Konzentration zwischen 0,5 und 5 % und die zweite thermochrome Zusammensetzung in einer Konzentration von 0,1 bis 5 Gew.-% des Gesamtgewichts des thermochromen Materials enthalten ist.

4. Enthaarungsmittel nach einem der vorhergehenden Ansprüche, bei dem die obere Temperaturgrenze, bei der die erste thermochrome Zusammensetzung ihre Farbe vollständig ändert und den maximalen Grad dieser Änderung erreicht, was zu einer homogenen Farbe des Enthaarungsmittels führt, mit der unteren Temperaturgrenze zusammenfällt, bei der die zweite thermochrome Zusammensetzung ihre Farbe zu ändern beginnt.

5. Enthaarungsmittel nach einem der vorhergehenden Ansprüche, bei dem die erste thermochrome Zusammensetzung ihre Farbe bei einer Temperatur zwischen 40 °C und 65 °C ändert, während die zweite thermochrome Zusammensetzung ihre Farbe bei einer Temperatur zwischen 65 °C und 100 °C ändert.

6. Enthaarungsmittel nach einem der vorhergehenden Ansprüche, das bei Raumtemperatur violett, bei einer Temperatur zwischen 60-65 °C rosa, wenn die Farbe der ersten thermochromen Zusammensetzung verschwindet, und weiß bei einer Temperatur von 65 °C oder mehr ist, wenn die Farbe des zweiten Enthaarungsmittels verschwindet.

7. Enthaarungsmittel nach einem der vorhergehenden Ansprüche, bei dem das thermochrome Material mindestens ein Polymer oder ein Copolymer enthält, um die Zusammensetzung des thermochromen Materials auf 100 Gew.-% zu vervollständigen.

8. Enthaarungsmittel nach dem vorhergehenden Anspruch, bei dem das Copolymer Polyethylen ist.

9. Enthaarungsmittel nach einem der vorhergehenden Ansprüche, das mindestens ein thermochromes Material in einer Menge zwischen 2 und 4 % des Gesamtgewichts des Mittels enthält.

10. Enthaarungsmittel nach einem der vorhergehenden Ansprüche, das ein heißes Wachs ist, das die folgende Zusammensetzung in Gewichtsprozent des Gesamtgewichts der Zusammensetzung haben kann:
- Glycerinharzat: 45-67 %
- Mikrokristallines Wachs: 10-30 %
- Lösungsvermittler: 5-8 %
- Extrakt: 2-6 %
- Bienenwachs: 2-6 %
- Gummi: 3-5 %
- Kosmetischer Inhaltsstoff: 1,0-2 %
- Essenz: 0.25-1.0 %
- Thermochromie-Farbstoffe: 2-4 %
- Erstes thermochromes Pigment: 0,1-8 Gewichtsprozent des Gesamtgewichts des Farbstoffs
- Zweites thermochromes Pigment: 0,05-8 Gewichtsprozent des Gesamtgewichts des Farbstoffs
- Polyethylen: csp. 100 Gewichtsprozent des Gesamtgewichts des Farbstoffs.

## Revendications

1. Composition dépilatoire **caractérisée en ce qu'**elle comprend au moins un matériau thermochrome contenant deux ou plus de deux compositions thermochromes réversibles, la première à une concentration comprise entre 0,1 et 8 %, et la deuxième à une concentration comprise entre 0,05 et 8 % en poids du poids total du matériau thermochrome, les deux étant sensibles à la chaleur à une température supérieure à 40°C mais différente pour chacune, de telle sorte que la première soit sensible à la chaleur dans une plage de température inférieure à celle de la deuxième.

2. Composition dépilatoire selon la revendication 1, qui est une cire dépilatoire tiède ou chaude.

3. Composition dépilatoire selon l'une quelconque des revendications précédentes, dans laquelle la première composition thermochrome réversible est contenue dans le matériau thermochrome à une concentration comprise entre 0,5 et 5 % et la deuxième composition thermochrome à une concentration de 0,1 % à -5 % en poids du poids total du matériau thermochrome.

4. Composition dépilatoire selon l'une quelconque des revendications précédentes, dans laquelle la limite de température supérieure à laquelle la première composition thermochrome change complètement de couleur et atteint le niveau maximal dudit changement, engendrant une couleur homogène dans la composition dépilatoire, coïncide avec la limite de température inférieure à laquelle la deuxième composition thermochrome commence à changer de couleur.

5. Composition dépilatoire selon l'une quelconque des revendications précédentes, dans laquelle la première composition thermochrome change de couleur à une température comprise entre 40°C et 65°C, tandis que la deuxième composition thermochrome change de couleur à une température comprise entre 65°C et 100°C.

6. Composition dépilatoire selon l'une quelconque des revendications précédentes, qui est pourpre à la température ambiante ; rose à une température comprise entre 60 et 65°C quand la couleur de la première composition thermochrome disparaît ; et blanche à une température égale ou supérieure à 65°C, quand la couleur de la deuxième composition dépilatoire disparaît.

7. Composition dépilatoire selon l'une quelconque des revendications précédentes, dans laquelle le matériau thermochrome contient au moins un polymère ou un copolymère, pour compléter la formulation du matériau thermochrome à 100 % en poids de celle-ci.

8. Composition dépilatoire selon la revendication précédente, dans laquelle le copolymère est le polyéthylène.

9. Composition dépilatoire selon l'une quelconque des revendications précédentes, contenant l'au moins un matériau thermochrome en une quantité comprise entre 2 % et 4 % du poids total de la composition.

10. Composition dépilatoire selon l'une quelconque des revendications précédentes, qui est une cire chaude qui peut avoir la formulation suivante, en pourcentages en poids du poids total de la composition :
- résinate de glycéryle à 45 à 67 %
- cire microcristalline : 10 à 30 %
- solubilisant : 5 à 8 %
- extrait : 2 à 6 %
- cire d'abeilles : 2 à 6 %
- caoutchouc : 3 à 5 %
- ingrédient cosmétique : 1,0 à 2 %
- huile essentielle : 0,25 à 1,0 %
- matériaux colorants thermochromes : 2 à 4 %
o premier pigment thermochrome : 0,1 à 8 % en poids du poids total du matériau colorant
o deuxième pigment thermochrome : 0,05 à 8 % en poids du poids total du matériau colorant
o polyéthylène : qsp 100 en poids du poids total du matériau colorant.
